# EUROPÄISCHE PATENTSCHRIFT

(11) **EP 0 667 125 B1**
(45) Veröffentlichungstag und Bekanntmachung des Hinweises auf die Patenterteilung: **16.08.2000**
(21) Anmeldenummer: 95250028.8
(22) Anmeldetag: 07.02.1995
(51) Int. Cl.: A61B 17/12, A61B 17/28

(54) **Medizinisches Gerät zum Injizieren und/oder Absaugen von Flüssigkeiten in ein bzw. aus einem Hohlorgan, insbesondere einem Gallengang**
Apparatus for injecting and/or aspirating fluids in /out of a hollow organ, especially a biliary duct
Appareil pour injecter et/ou aspirer des fluides dans un/d'un organe creux, en particulier un canal biliaire

(30) Priorität: 09.02.1994 DE 4404781
(43) Veröffentlichungstag der Anmeldung: 16.08.1995
(73) Patentinhaber: Otten, Gert, Prof., Dr., 27619 Schiffdorf (DE)
(72) Erfinder: Otten, Gert, Prof. Dr. med., D-27619 Schiffdorf (DE); Lindeke, Carsten, D-12459 Berlin (DE)
(74) Vertreter: Porth, Hans-Joachim

(56) Entgegenhaltungen:
- EP-A- 0 519 590
- FR-A- 2 644 056
- US-A- 4 484 911
- US-A- 4 792 330
- US-A- 5 131 379
- US-A- 5 350 384

## Beschreibung

Die Erfindung betrifft ein medizinisches Gerät nach dem Oberbegriff des Patentanspruchs 1.

Ein derartiges Geräte ist bereits bekannt (US-A-4 484 911). Dieses Gerät weist eine Kanüle auf, der eine Spanneinrichtung zugeordnet ist. Die Spanneinrichtung weist zwei zueinander schwenkbewegliche profilierte längliche Backen auf, zwischen denen nahezu koaxial die Kanüle angeordnet ist. Die Spanneinrichtung dient dazu, die in ein Hohlorgan eingeführte Kanüle im Bereich zwischen einer Eintrittsöffnung des Hohlorgans und einer Austrittsöffnung der Kanüle zu umschließen und dabei das Gewebe des Hohlorgans gegen die Kanüle zu drücken. Hierdurch soll sichergestellt werden, daß während des Injizierens oder Absaugens ein Flüssigkeitsdurchtritt in diesem Bereich über den Ringspalt zwischen Hohlorgan und Kanüle ausgeschlossen wird.

Der Erfindung liegt die Aufgabe zugrunde, ein medizinisches Gerät der eingangs erwähnten Art zu schaffen, welches ein exaktes und verlustfreies Injizieren bzw. Absaugen der Flüssigkeit gestattet, umfassend anwendbar, leicht handhabbar und zudem mit geringem Aufwand herstellbar ist.

Erfindungsgemäß wird die Aufgabe dadurch gelöst, daß das Gewebe des Hohlorgans im Bereich eines abgewinkelten Einführungsteils des freien Endes gegen die Kanüle abdichtbar ist. Wegen des abgewinkelten Einführungsteils ist die Anordnung des Einführungsteils innerhalb des Gallengangs wesentlich erleichtert und eine sichere Abdichtung im Bereich des Einführungsteils ermöglicht, folglich innerhalb einer längeren Abschnitts des Gallengangs. Durch die abgewinkelte Anordnung des Einführungsteils wird die seitliche Einführung und Plazierung der Kanüle wie auch der Spanneinrichtung begünstigt.

In weiterer Ausgestaltung der Erfindung ist die Spanneinrichtung eine Spannzange, deren Backen derart relativbeweglich sind, daß das Gewebe bzw. die Kanüle von einem Zangenmaul in der Spannstellung der Spannzange fest umschlossen und in deren Ausgangsstellung freigegeben ist. Allein durch die Bewegung der Spannbacken und somit in einfachster Weise kann die abgedichtete Anordnung der Kanüle in dem Gallengang gewährleistet werden.

In vorteilhafter Ausgestaltung endet die eine Backe in zwei gabelförmig angeordneten Zinken und die andere in einer Zinke. Durch diese Zinken wird das Zangenmaul gebildet, wobei in der Spannstellung der Spannzange die Zinke der einen Backe zwischen den beiden Zinken der anderen Backe angeordnet ist, die Zinken gewissermaßen miteinander verkämmt sind. Wegen des Ineinanderkämmens der recht schmalen Zinken ist eine zentrierte Fixierung des Gewebes nahe der Kanüle gegeben, unabhängig von der Form oder Dicke des Gewebes bzw. einer unsymmetrischen Anordnung desselben um die Kanüle herum.

Zweckmäßig ist es, daß sich mindestens die Zinken in Richtung der Kanüle etwa bogenförmig erstrecken und dadurch das Gewebe in Radialrichtung des Einführungsteils und annähernd gleichmäßig am Kanülenumfang gegen die Kanüle angepreßt wird. Die sichere Fixierung des Gewebes und Abdichtung erfordert lediglich einen kurzen Längsabschnitt des Einführungsteils, wodurch die Abdichtung begünstigt wird.

Zur besseren Fixierung des Gewebes kann mindestens eine mit dem Gewebe in Kontakt tretende Zinkenfläche mit einer Profilierung versehen sein oder eine aufgerauhte Oberfläche aufweisen.

Die Flüssigkeit wird über einen mit der Kanüle verbundenen Anschluß ein- bzw. abgeleitet. Dieser tritt vorzugsweise aus dem Mittelbereich des Rohrschafts aus, so daß Zu- bzw. Ableitungen leicht anschließbar sind.

In weiterer Ausgestaltung der Erfindung erstreckt sich neben der Kanüle ein Grundkörper der Spannzange. Er tritt im wesentlichen parallel zur Kanüle und wie diese exzentrisch aus einem Kopf eines Rohrschafts aus. An den Grundkörper sind gegenläufig schwenkbeweglich die Backen der Spannzange derart angelenkt, daß das Zangenmaul die Kanüle bzw. das Gewebe stets im Bereich des Einführungsteils der Kanüle umgibt. Somit erfolgt die Abdichtung zwangsweise zwischen der zuvor geschaffenen Eintrittsöffnung des Hohlorgans und der Austrittsöffnung der Kanüle. Flüssigkeit kann in das Hohlorgan injiziert bzw. aus diesem abgesaugt werden, ohne das Verluste wegen Undichtheit zwischen Gewebe und Kanüle auftreten.

Weitere Merkmale der Erfindung und deren Vorteile ergeben sich aus den übrigen Ansprüchen und der Beschreibung.

Die Erfindung soll nachfolgend an einem Ausführungsbeispiel näher erläutert werden. In der zugehörigen Zeichnung zeigen:
- Fig. 1: eine Seitenansicht des medizinischen Geräts schematisch dargestellt,
- Fig. 2: die Draufsicht des Geräts nach Fig. 1 mit in Ausgangsstellung angeordneter Spannzange, schematisch dargestellt und
- Fig. 3: die teilweise Draufsicht nach Fig. 2 mit in Spannstellung angeordneter Spannzange.

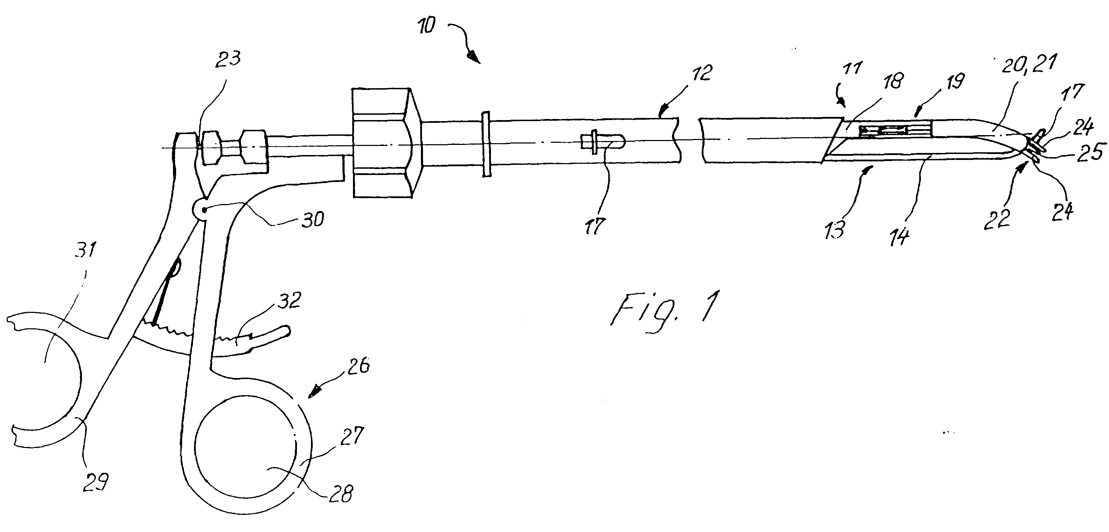

Ein medizinisches Gerät 10 (Fig. 1) zum Injizieren und/oder Absaugen von Flüssigkeiten in ein oder aus einem Hohlorgan, beispielsweise einem Gallengang, ist als Rohrschaftinstrument ausgebildet. Einerseits eines Rohrschafts 12 tritt aus einem Kopf 11 desselben eine Kanüle 13 aus. Diese erstreckt sich im wesentlichen parallel und exzentrisch vom Rohrschaft 12 sowie von dessen unterer Peripherie. Ein freies Ende 14 der Kanüle 13 ist nahe einer Austrittsöffnung 15 in Richtung der Symmetrielinie des Rohrschafts 12 abgewinkelt, wodurch eine Einführungsteil 16 der Kanüle 13 gebildet ist. Die Kanüle 13 kann somit über eine zuvor geschaffene Eintrittsöffnung des Gallengangs seitlich in diesen eingeführt und etwa koaxial innerhalb des Gallengangs mittels des Einführungsteils 16 sicher plaziert werden.

In einem Mittelbereich des Rohrschafts 12 tritt aus diesem ein Anschluß 17 aus, der mit der Kanüle 13 verbunden ist, so daß über den Anschluß 17 Flüssigkeit, beispielsweise Kontrastmittel, in den Gallengang injiziert und ggf. nach dem Röntgen der Galle wieder abgesaugt werden kann. In gleicher Weise ist es möglich, Gallensaft abzusaugen.

Aus dem Kopf 11 des Rohrschafts 12 tritt an der Kanüle 13 gegenüberliegenden Peripherie und etwa parallel zur Kanüle 13 auch ein Grundkörper 18 einer als Spannzange 19 ausgebildeten Spanneinrichtung aus. Die Spannzange 19 dient dazu, das Gewebe des Gallengangs, das bei eingeführter Kanüle 13 um deren Einführungsteil 16 herum angeordnet ist, fest gegen letzteres anzupressen und damit eine Abdichtung zu erreichen, die einen Flüssigkeitsdurchtritt zwischen diesem Gewebe und der Kanüle 13 ausschließt. Die Abdichtung ist dadurch gegeben, daß das Gewebe zwischen der Austrittsöffnung 17 der Kanüle 13 und der Eintrittsöffnung gegen die Kanüle 13 angepreßt wird. Hierdurch ist unter anderem die eindeutige und beweisfähige Erkennung des zur Gallenblase führenden Gallengangs möglich, was für den weiteren Verlauf eines chirurgischen Eingriffs von entscheidender Bedeutung ist.

Die Spannzange 19 weist zwei Backen 20, 21 auf, die gegenläufig schwenkbeweglich an dem Grundkörper 18 angelenkt sind. Die Schwenkbewegung wird mittels eines Gestänges 23 eingeleitet, welches sich entlang des Grundkörpers 18 erstreckt und an der den Backen 20, 21 gegenüberliegenden Seite aus dem Grundkörper 18 bzw. dem Rohrschaft 12 austritt. Die Backen 20, 21 sind derart angeordnet, daß die Kanüle 13 bzw. das dort befindliche Gewebe im Bereich des Einführungsteils 16 von einem Zangenmaul 22 der Spannzange 19 in Spannstellung fest umschlossen oder in Ausgangsstellung freigegeben ist.

Das Zangenmaul 22 ist durch zwei gabelförmig angeordnete Zinken 24, in denen die eine Backe 20 endet, und eine Zinke 25, in der die andere Backe 21 endet, gebildet. In der Spannstellung der Spannzange 19 ist die Zinke 25 zwischen den Zinken 24 angeordnet, so daä diese gewissermaßen ineinander verkämmt sind. Dabei wird das Einführungsteil 16 der Kanüle 13 und das umgebene Gewebe zentriert und fest umschlossen. Die Zentrierung erfolgt zwischen den Zinken 24, 25 und unweit des Bereichs der Backe 20, von dem sich die Zinken 24 gabelförmig erstrecken.

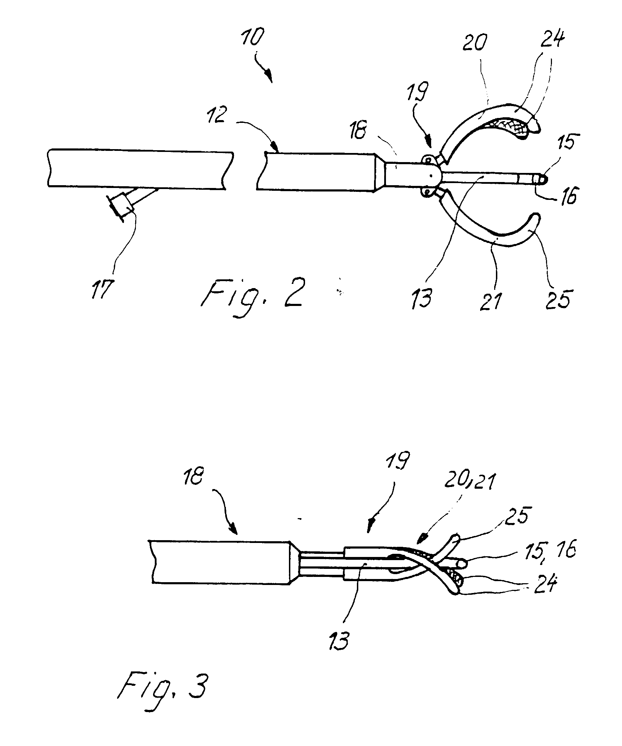

Die Backen 20, 21 sind wie auch deren Zinken 24, 25 in ihrer Bewegungsebene bogenförmig ausgebildet, wie insbesondere aus Fig. 2 ersichtlich. Dabei ist die eine Zinke 24 der Backe 20 etwas vorgeschoben dargestellt, um deren gabelförmige Ausbildung besser zu veranschaulichen. Die Zinken 24, 25 sind somit wegen der Gestaltung der Backen 20, 21 und der Anlenkung an den Grundkörper 18 etwa wie eine Kneifzange schwenkbeweglich.

Die Spannzange 19 und deren Backen 20, 21 und Zinken 24, 25 haben in Radialrichtung des Rohrschafts 12, zumindest sofern sich die Spannzange 19 in der Spannstellung befindet, eine geringe Erstreckung, die kleiner ist als der Innendurchmesser eines im Falle eines laparoskopischen Eingriffs verwendeten Trokars, so daß dieses Gerät auch bei einem derartigen Eingriff verwendet werden kann.

Die Zinken 24, 25 sind außerdem zusätzlich in Richtung des Einführungsteils 16 der Kanüle 13 bogenförmig umgelenkt. Diese Umlenkung entspricht etwa der Abwinklung der Kanüle 13, erfolgt aber in Richtung der Kanüle 13 und damit gewissermaßen in entgegengesetzter Richtung zur Abwinklung. Wegen dieser Ausbildung der Backen 20, 21 und Zinken 24, 25 ist sichergestellt, daß die Kanüle 13 und das Gewebe des Hohlorgans etwa in Radialrichtung des Einführungsteils 16 und annähernd gleichmäßig am Umfang des letzteren angepreßt werden. Eine sichere Abdichtung ist somit unabhängig von Form und Dicke des Gewebes stets gegeben.

Die Oberfläche der Zinken 24, 25, zumindest der Teil, der zur Anlage an das Gewebe gelangt, kann auch mit einer Profilierung, beispielsweise einer Rändelung versehen sein. Hierdurch kann die Fixierung des Gewebes innerhalb des Zangenmauls 22 zusätzlich verbessert werden.

An dem den Backen 20, 21 gegenüberliegenden Ende des dort aus dem Rohrschaft 12 austretenden Grundkörpers 18 der Spannzange 19 ist ein scherenförmiger Griff 26 angeordnet, der sowohl zur Handhabung des medizinischen Geräts 10 wie auch Betätigung der Backen 20, 21 der Spannzange 19 dient. Eine Griffhälfte 27 ist auf der einer Fingeraufnahme 28 abgewandten Seite fest mit dem diesseitigen Ende des Grundkörpers 18 verbunden. Eine andere Griffhälfte 29 ist dazu um eine Achse 30 schwenkbeweglich. An der einer Fingeraufnahme 31 gegenüberliegenden Seite ist an die Griffhälfte 29 das Gestänge 23 angelenkt. Bei einer Schwenkbewegung der Griffhälften 27, 29 um die Achse 30 wird das Gestänge 23 entlang des Grundkörpers 18 bzw. Rohrschafts 12 in Axialrichtung verschoben, woraufhin die gegenläufige Schwenkbewegung der Backen 20, 21 von der Ausgangsstellung in die Spannstellung und umgekehrt erfolgen kann. Zur Realisierung dieser Relativbewegung der Backen 20, 21 sind diese wie auch ein entsprechender Bereich des Gestänges 23 über einen Scherenhebeltrieb verbunden. Sie können aber auch partiell als korrespondierende Elemente eines Zahnstangentriebs oder dgl. ausgebildet sein.

Schließlich ist den Griffhälften 27, 29 eine an sich bekannte Halteeinrichtung 32 zugeordnet, mit der die Griffhälften 27. 29 und somit auch die Backen 20, 21 in einer der Spannstellung bzw. Ausgangsstellung der Spannzange 19 entsprechenden Position fixierbar sind.

### Bezugszeichenaufstellung

- 10: Gerät
- 11: Kopf
- 12: Rohrschaft
- 13: Kanüle
- 14: Ende
- 15: Austrittsöffnung
- 16: Einführungsteil
- 17: Anschluß
- 18: Grundkörper
- 19: Spannzange
- 20: Backe
- 21: Backe
- 22: Zangenmaul
- 23: Gestänge
- 24: Zinke
- 25: Zinke
- 26: Griff
- 27: Griffhälfte
- 28: Fingeraufnahme
- 29: Griffhälfte
- 30: Achse
- 31: Fingeraufnahme
- 32: Halteeinrichtung

## Patentansprüche

1. Medizinisches Gerät (10) zum Injizieren und/oder Absaugen von Flüssigkeit in ein bzw. aus einem Hohlorgan, insbesondere einem Gallengang, mit einer über eine Eintrittsöffnung in das Hohlorgan einführbaren Kanüle (13), wobei der Kanüle (13) eine Spanneinrichtung zugeordnet ist, mit der das Gewebe des Hohlorgans zumindest zwischen der Eintrittsöffnung und einer Austrittsöffnung (15) der Kanüle (13) derart umschließbar ist, daß ein Flüssigkeitsdurchtritt zwischen dem Gewebe und der Kanüle (13) ausgeschlossen ist und die Kanüle (13) ein freies Ende (14) aufweist, dadurch gekennzeichnet, daß das Gewebe im Bereich eines abgewinkelten Einführungsteils (16) des freien Endes (14) gegen die Kanüle (13) abdichtbar ist.

2. Medizinisches Gerät nach Anspruch 1, dadurch gekennzeichnet, daß die Spanneinrichtung eine Spannzange (19) ist, deren Backen derart relativbeweglich sind, daß die Kanüle (13) bzw. das Gewebe von einem Zangenmaul (22) in Spannstellung umschließbar oder in Ausgangsstellung freigebbar ist.

3. Medizinisches Gerät nach Anspruch 2, dadurch gekennzeichnet, daß das Zangenmaul (22) durch mindestens eine Zinke (24, 25) jeder der Backen (20, 21) gebildet ist und die Zinken (24, 25) ineinander kämmend anordenbar sind.

4. Medizinisches Gerät nach Anspruch 2 und 3, dadurch gekennzeichnet, daß die eine Backe (20) einerseits in zwei gabelförmig angeordneten Zinken (24) und die andere Backe (21) einer Zinke (25) endet und letztere in Spannstellung in die gabelförmig angeordneten Zinken (24) einkämmt.

5. Medizinisches Gerät nach Anspruch 3 und 4, dadurch gekennzeichnet, daß sich zumindest die Zinken (24, 25) derart in Richtung der Kanüle (13) etwa bogenförmig erstrecken, daß die Kanüle (13) bzw. das Gewebe in Radialrichtung des Einführungsteils (16) und annähernd gleichmäßig um den Kanülenumfang umschließbar ist.

6. Medizinisches Gerät nach einem oder mehreren der Ansprüche 3 bis 5, dadurch gekennzeichnet, daß das Einführungsteil (16) des freien Endes (14) der Kanüle (13) in Richtung der Symmetrieachse eines Rohrschafts (12) abgewinkelt ist und die Zinken (24, 25) in Richtung des Einführungsteils (16) umgelenkt sind und die Umlenkung etwa der Abwinklung der Kanüle (13) in entgegengesetzter Richtung entspricht.

7. Medizinisches Gerät nach Anspruch 6, dadurch gekennzeichnet, daß die Kanüle (13) mit dem freien Ende (14) aus einem Kopf (11) des Rohrschafts (12) austritt und sich parallel und exzentrisch zum Rohrschaft (12) erstreckt.

8. Medizinisches Gerät nach einem oder mehreren der Ansprüche 6 und 7, dadurch gekennzeichnet, daß sich neben der Kanüle (13) ein Grundkörper (18) der Spannzange (19) erstreckt, dieser aus dem Kopf (11) des Rohrschafts (12) austritt und an ihm die Backen (20, 21) der Spannzange (19) gegenläufig schwenkbar angelenkt sind.

9. Medizinisches Gerät nach Anspruch 8, dadurch gekennzeichnet, daß eine Griffhälfte (27) eines scherenförmigen Griffs (26) fest mit dem Grundkörper (18) der Spannzange (19) verbunden und an eine dazu schwenkbewegliche andere Griffhälfte (29) ein Gestänge (23) zur Betätigung der Backen (20, 21) der Spannzange (19) angelenkt ist.

## Revendications

1. Appareil médical (10) destiné à injecter et/ou à aspirer du liquide dans un organe creux et hors de celui-ci respectivement, notamment d'un canal biliaire, muni d'une canule (13) susceptible d'être introduite dans l'organe creux par l'intermédiaire d'une ouverture d'entrée, un dispositif de serrage étant associé à la canule (13), dispositif à l'aide duquel les tissus de l'organe creux sont susceptibles d'être entourés au moins entre l'ouverture d'entrée et une ouverture de sortie (15) de la canule (13) de façon à ce qu'une pénétration du liquide entre les tissus et la canule (13) soit impossible, et la canule (13) présentant une extrémité libre (14), **caractérisé par le fait que** les tissus sont susceptibles d'être calfeutrés par rapport à la canule (13) dans le domaine d'un élément d'introduction (16) coudé de l'extrémité libre (14).

2. Appareil médical selon la revendication 1, **caractérisé par le fait que** le dispositif de serrage est une pince de serrage (19) dont les mâchoires sont susceptibles de se déplacer l'une par rapport à l'autre de telle façon que la canule (13) et les tissus respectivement sont susceptibles d'être entourés en position de serrage par la gueule d'une pince (22) ou sont susceptibles d'être débloqués en position de sortie.

3. Appareil médical selon la revendication 2, **caractérisé par le fait que** la gueule de la pince (22) est formée d'au moins une dent (24, 25) de chacune des mâchoires (20, 21) et que les dents (24, 25) sont susceptibles de se disposer les unes dans les autres à la façon d'un engrenage.

4. Appareil médical selon la revendication 2 et 3, **caractérisé par le fait que** l'une des mâchoires (20) débouche d'un côté dans deux dents disposées en forme de fourche (24) et dans l'autre mâchoire (21) d'une dent (25) et que cette dernière s'engrène en position de serrage dans les dents disposées en forme de fourche (24).

5. Appareil médical selon la revendication 3 et 4, **caractérisé par le fait qu'**au moins les dents (24, 25) s'étirent à peu près en forme d'arc dans le sens de la canule (13) de telle façon que la canule (13) et les tissus respectivement sont susceptibles d'être entourés dans le sens radial de l'élément d'introduction (16) et à peu près uniformément autour de la circonférence de la canule.

6. Appareil médical selon l'une ou plusieurs des revendications 3 à 5, **caractérisé par le fait que** l'élément d'introduction (16) de l'extrémité libre (14) de la canule (13) est coudé dans le sens de l'axe de symétrie d'une tige tubulaire (12) et les dents (24, 25) sont retournées dans le sens de l'élément d'introduction (16) et le retournement correspond à peu près au pliage de la canule (13) dans le sens opposé.

7. Appareil médical selon la revendication 6, **caractérisé par le fait que** la canule (13) émerge avec l'extrémité libre (14) d'une tête (11) de la tige tubulaire (12) et s'étire parallèlement et excentriquement par rapport à la tige tubulaire (12).

8. Appareil médical selon l'une ou plusieurs des revendications 6 et 7, **caractérisé par le fait qu'**un corps de base (18) de la pince de serrage (19) s'étire à côté de la canule (13), que celui-ci émerge de la tête (11) de la tige tubulaire (12) et que les mâchoires (20, 21) de la pince de serrage (19) sont articulées à celui-ci de façon susceptible de pivoter l'une par rapport à l'autre.

9. Appareil médical selon la revendication 8, **caractérisé par le fait qu'**une moitié de manche (27) d'un manche en forme de ciseaux (26) est reliée fixement au corps de base (18) de la pince de serrage (19) et qu'un montant (23) destiné à fixer les mâchoires (20, 21) de la pince de serrage (19) est articulé à l'autre moitié de manche (29) susceptible de se déplacer en pivotant par rapport à la première.

## Claims

1. Medical instrument (10) for the injection and/or withdrawal of fluids into or out of a hollow organ, especially the bile duct, with a cannula (13) which can be inserted into the hollow organ through an entry opening. A tensor assemblage is attached to the cannula (13) with which the tissue of the hollow organ between the entry into the organ and the opening (15) of the cannula (13) can be closed in order to block the passage of fluid between the tissue and the cannula (13). The cannula (13) has a free end (14) which makes it possible to seal the tissue against the cannula with an angled insertion part (16) of the free end (14).

2. Medical instrument in Claim 1, characterized by a tensor assemblage, or forceps (19), the jaws of which are moveable in relation to one another, so that the cannula (13) or the tissue can be held closed or held open in the release position by the mouth (22) of the forceps.

3. Medical instrument in Claim 2, where the mouth (22) of the forceps is made up of at least one prong (24, 25) of each of the jaws (20, 21) and the prongs (24, 25) fit together in a comb-like manner.

4. Medical instrument in Claims 2 and 3, characterized by one jaw (20) which ends in two fork-shaped prongs (24) and another jaw (21) which terminates in a prong. The latter fits with the fork-shaped prongs (24) in a comb-like manner.

5. Medical instrument in Claims 3 and 4, characterized by prongs (24, 25) which extend toward the cannula (13) in roughly the shape of an arch. With these, the cannula (13) or the tissue can be closed along the radius of the insertion piece (16) and fastened evenly around the circumference of the cannula.

6. Medical instrument in one or more of Claims 3 to 5, characterized by an insertion piece (16) of the free end (14) of the cannula (13) which is angled toward the axis of symmetry of a tube or barrel (12). The prongs (24, 25) are turned toward the insertion piece (16), and this turn or bend roughly corresponds, in the opposite direction, to the angle of the cannula (13).

7. Medical instrument in Claim 6, characterized by the cannula (13) extending with the free end (14) out of a head (11) of the barrel (12), parallel and eccentrically to the barrel (12).

8. Medical instrument in one or more of Claims 6 and 7, in which the main section (18) of the forceps (19) is extended along with the cannula (13), coming out of the head (11) of the barrel (12). On this section, the jaws (20, 21) of the forceps (19) are attached to one another with a swivel attachment.

9. Medical instrument in Claim 8, characterized by the fact that one half (27) of the handle of a scissor-shaped grip (26) is securely fastened to the main part (18) of the forceps (19) and a second half (29) of the handle, which can be pivoted against the first half (29), is connected to a rod (23) for operating the jaws (20, 21) of the forceps (19).
